# EUROPÄISCHE PATENTANMELDUNG

(11) **EP 1 034 754 A1**
(43) Veröffentlichungstag der Anmeldung: **13.09.2000**
(21) Anmeldenummer: 00810120.6
(22) Anmeldetag: 11.02.2000
(51) Int. Cl.: A61F 2/34

(54) **Bausatz einer Aussenschale für eine künstliche Hüftgelenpfanne**

(30) Priorität: 10.03.1999 EP 99810210
(71) Anmelder: Sulzer Orthopedics Ltd., 6340 Baar (CH)
(72) Erfinder: Willert, Hans-Georg, Prof. Dr. med., 37075 Göttingen (DE); Bider, Kurt, 8406 Winterthur (CH)
(74) Vertreter: Sulzer Management AG

(57) **Zusammenfassung**

Bausatz einer Aussenschale (1) für eine künstliche Hüftgelenkpfanne, umfassend ein schalenförmiges Aussenteil (2) sowie ein als Lasche (3, 4), Dorn (5) oder Haken (6) ausgestaltetes Verankerungshilfsmittel (7), wobei das schalenförmige Aussenteil (2) an dessen Äquator eine Stirnfläche (2a) mit Bohrungen (2b) aufweist, und wobei jedes Verankerungshilfsmittel (7) ein Befestigungsteil (3a, 4a, 5a) aufweist, welches derart bezüglich der Stirnfläche (2a) angepasst ausgestaltet ist, dass das Befestigungsteil (3a, 4a, 5a) mittels einer in die Bohrung (2b) eingreifenden Schraube (6) am schalenförmigen Aussenteil (2) befestigbar ist.

## Beschreibung

Die Erfindung betrifft einen Bausatz einer Aussenschale für eine künstliche Hüftgelenkpfanne gemäss dem Oberbegriff von Anspruch 1.

Aus der Druckschrift EP-A-0 563 503 ist eine Aussenschale für eine künstliche Hüftgelenkpfanne bekannt, welche aus einer halbkugelförmigen Schale sowie an deren äquatorialem Rand vorstehend angeordnete Laschenteile besteht. Eine derartige Aussenschale wird beispielsweise verwendet wenn das Acetabulum an der zu tragenden Stelle beschädigt oder stark degeneriert ist, was insbesondere bei Revisionen von implantierten künstlichen Gelenken häufig der Fall ist. Die Laschenteile werden während dem Implantieren durch plastische Deformation dem Verlauf des Knochens "Os ilium" beziehungsweise des Knochens "Os ischii" angepasst und danach mittels Knochenschrauben in einem tragfähigen Bereich verankert, sodass die Aussenschale fest am Beckenknochen befestigt ist.

Nachteilig ist die Tatsache, dass eine Vielzahl unterschiedlich grosser Aussenschalen erforderlich ist. Nachteilig an der bekannten Aussenschale ist zudem die Tatsache, dass zum Implantieren eine relativ grosse Öffnung am Operationsfeld erforderlich ist. Zudem ist das Anpassen des Verlaufs der Laschenteile an den Verlauf des Beckenknochens schwierig und umständlich.

Es ist Aufgabe der vorliegenden Erfindung eine Aussenschale vorzuschlagen, welche kostengünstiger ist und einfacher zu implantieren ist.

Diese Aufgabe wird gelöst mit einem Bausatz einer Aussenschale aufweisend die Merkmale von Anspruch 1.

Die Unteransprüche 2 bis 9 beziehen sich auf weitere, vorteilhafte Ausgestaltungen der Aussenschale.

Die Aufgabe wird insbesondere gelöst mit einem Bausatz für eine Aussenschale umfassend ein schalenförmiges Aussenteil sowie ein als Lasche, Dorn oder Haken ausgestaltetes Verankerungshilfsmittel, wobei das schalenförmige Aussenteil an dessen Äquator eine Stirnfläche mit Bohrungen aufweist, und wobei jedes Verankerungshilfsmittel ein Befestigungsteil aufweist, welches derart bezüglich der Stirnfläche angepasst ausgestaltet ist, dass das Befestigungsteil mittels in die Bohrung eingreifenden Schrauben am schalenförmigen Aussenteil befestigbar ist.

Der modulare Aufbau der erfindungsgemässen Aussenschale weist den Vorteil auf, dass der Bausatz eine Mehrzahl von unterschiedlich geformten, schalenförmigen Aussenteilen und/oder Verankerungshilfsmittel umfasst, sodass es dem Chirurgen interoperativ freigestellt ist zu entscheiden, welcher schalenförmige Aussenteil und/oder welche Verankerungshilfsmittel entsprechend dem Zustand und der Anatomie des Beckenknochens zur Verankerung der Aussenschale am besten geeignet sind. Da die Aussenschale im Operationsfeld in situ zusammengesetzt wird kann die Öffnung des Operationsfeldes klein gehalten werden, was sich beispielsweise schonend auf die Weichteile auswirkt. Nach dem Einführen des schalenförmigen Aussenteiles kann eine geeignete Lasche ausgewählt und im Operationsfeld an den Beckenknochen angelegt werden. Die Lasche weist vorteilhafterweise einen dem anatomischen Verlauf des Beckenknochens vorgeformten Verlauf auf. Sollten sich beim Anpassen der Lasche an den Beckenknochen ergeben, dass deren gegenseitige Form noch nicht optimal übereinstimmt, so kann die Lasche wieder aus dem Operationsfeld entfernt und ausserhalb des Operationsfeldes mit geeigneten Werkzeugen nachgeformt werden. Sofern erforderlich kann der Vorgang des Anlegens der Lasche an den Beckenknochen und das Nachformen mehrmals wiederholt werden. Sobald die Lasche die anatomisch gewünschte Form aufweist kann die Lasche mit Hilfe einer Schraube fest mit dem schalenförmigen Aussenteil verbunden werden. Die optimale Auswahl der Laschenform, der Laschengrösse und deren Art der Verankerung im Beckenknochen ist zur sicheren, langfristigen Verankerung der Aussenschale insbesondere dann von Bedeutung, wenn der Beckenknochen grössere Defekte wie Löcher aufweist. Zudem kann auch das schalenförmige Aussenteil entsprechend dem Defekt des Beckenknochens gewählt werden. Es stehen beispielsweise runde oder oval ausgestaltete Aussenteile zur Auswahl zur Verfügung. Es erweist sich beispielsweise als Vorteilhaft bei einem grossen Loch im Beckenknochen ein entsprechend grosses Aussenteil zu wählen, um das Loch mit dem Implantat zu füllen, was den Knochen stabilisiert.

Sollte sich während dem Implantieren ergeben, dass zur Fixierung des Aussenteils die Lasche nicht geeignet ist oder zusätzliche Verankerungshilfen erforderlich sind, so können als weitere Verankerungshilfe beispielsweise ein Dorn oder ein Haken verwendet werden.

Die Erfindung wird an Hand mehrerer Ausführungsbeispiele beschrieben. Es zeigen:
- Fig. 1: eine perspektivische Ansicht einer zusammengestellten Aussenschale;
- Fig. 2: eine perspektivische Ansicht einer weiteren Aussenschale;
- Fig. 3: eine Teilansicht eines Querschnittes durch die Aussenschale.

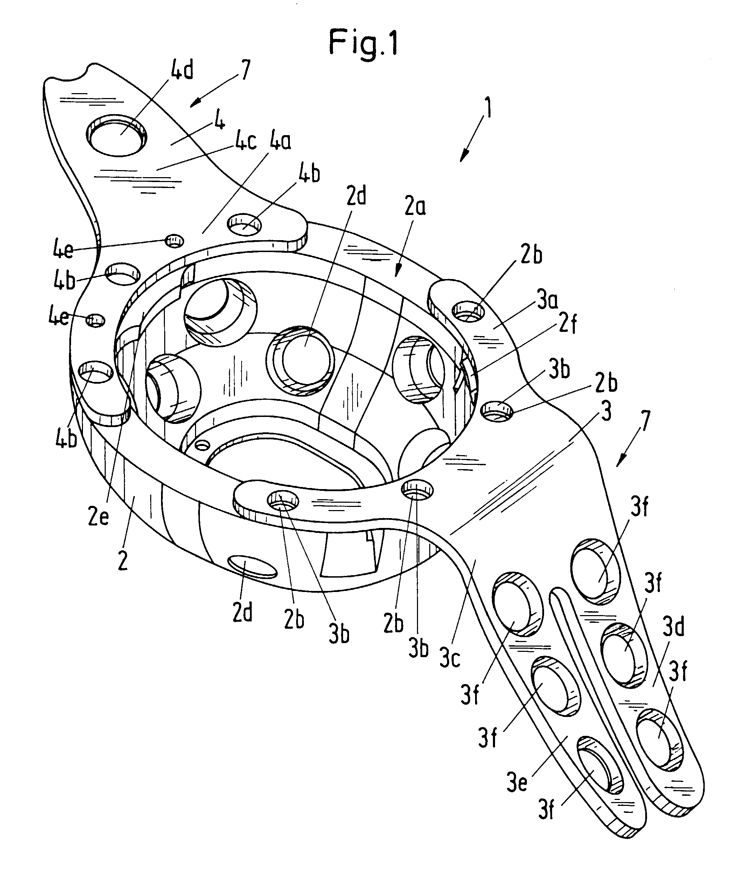

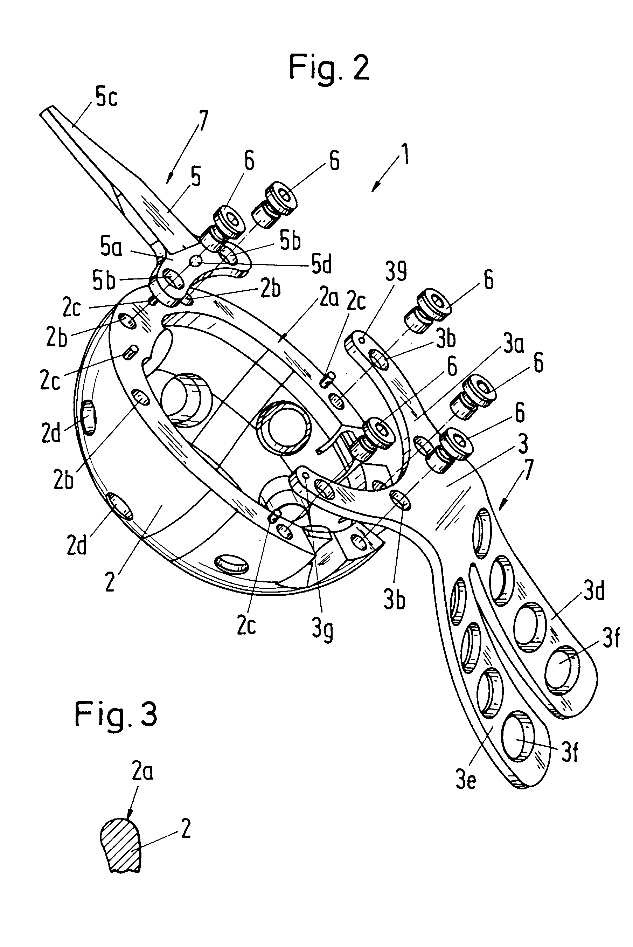

Fig. 1 zeigt eine Aussenschale 1, welche aus einem schalenförmigen Aussenteil 2 sowie zwei mit dem Aussenteil 2 lösbar verbundene, als Laschen 3, 4 ausgestaltete Verankerungshilfsmittel 7 besteht. Das schalenförmige Aussenteil 2 weist an dessen Äquator eine Stirnfläche 2a auf, welche im dargestellten Ausführungsbeispiel als ebene Fläche ausgestaltet ist. Die Stirnfläche 2a weist mehrere über deren Umfang verteilt angeordnete Bohrungen 2b auf, welche ein Innengewinde zur Aufnahme einer Schraube 6 aufweisen. Die Laschen 3, 4 umfassen je ein Befestigungsteil 3a, 4a, welches zur Befestigung an der Stirnfläche 2a bestimmt ist, wobei das Befestigungsteil 3a, 4a als Bohrung ausgestaltete Durchbrechungen 3b aufweist, welche deckungsgleich zu den Bohrungen 2b angeordnet sind. Das Befestigungsteil 3a, 4a ist, wie in Fig. 2 dargestellt, mittels Schrauben 6, welche durch die Durchbrechung 3b verlaufend in die Bohrung 2b eingreifen, fest mit dem schalenförmigen Aussenteil 2 verbindbar. Fig. 1 zeigt das schalenförmige Aussenteil 2 sowie die Laschen 3, 4 in der fest verbundenen Lage, wobei der Übersichtlichkeit halber die Schrauben 6 nicht dargestellt sind.

Die Lasche 3 ist einstückig ausgestaltet und besteht aus dem Befestigungsteil 3a, einem daran anschliessenden Übergangsteil 3c, welcher in zwei zungenförmige, im wesentlichen parallel zueinander verlaufende Laschenteile 3d, 3e mündet. Die Laschenteile 3d, 3e weisen Durchbrechungen 3f auf, durch welche Knochenschrauben zur Verankerung im Beckenknochen einführbar sind. Die Lasche 3 ist derart vorgeformt, dass zumindest die Laschenteile 3d, 3e ungefähr einen entsprechend der Anatomie des Beckenknochens angepassten Verlauf aufweisen. Die Lasche 4 ist ebenfalls einstückig ausgebildet und besteht aus einem Befestigungsteil 4a, welches Durchbrechungen 4b zur Aufnahme einer Schraube 6 sowie Durchbrechungen 4e zur Aufnahme eines Nocken 2c aufweist, sowie einem Laschenteil 4c mit Durchbrechung 4d für eine Knochenschraube. Die Lasche 4 ist eben verlaufend ausgestaltet.

Das Aussenteil 2 ist in der dargestellten Ausführungsform oval ausgestaltet. Das Aussenteil 2 weist Durchbrechungen 2d auf, durch welche gegebenenfalls Knochenschrauben zur Verankerung im Beckenknochen einführbar sind. Das Aussenteil 2 umfasst im dargestellten Ausführungsbeispiel zudem ein Nocken 2e sowie Schnappteile 2f, welche zum Halten einer nicht dargestellten Innenschale dienen.

Fig. 2 zeigt ein weiteres Ausführungsbeispiel einer Aussenschale 1 mit Verankerungshilfsmitteln 7 sowie Schrauben 6. Identische Bezugszeichennummern bezeichnen dieselben, bereits in Fig. 1 erläuterten Gegenstände. An der Stirnfläche 2a sind nebst den Bohrungen 2b zusätzlich vorstehende Nocken 2c angeordnet. Das Befestigungsteil 3a der Lasche 3 weist nebst den für die Schrauben 6 vorgesehenen Durchbrechungen 3b zudem zwei Bohrungen 3g auf, welche derart angeordnet sind, dass sie je einen Nocken 2c umschliessen, wenn die Lasche 3 auf der Stirnfläche 2a aufliegt. Die Nocken 2c dienen einerseits als Hilfe zum richtigen Positionieren der Lasche 3 während dem Auflegen auf die Stirnfläche 2a. Zudem dienen die Nocken 2c zur Aufnahme von durch die Lasche 3 bewirkten Schubspannungen. Eine weitere Ausführungsform eines Verankerungsmittels 7 ist als ein Dorn 5 ausgestaltet, welcher aus einem Befestigungsteil 5a sowie einem Dornteil 5c besteht. Das Befestigungsteil 5a weist zwei Durchbrechungen 5b auf, durch welche je eine Schraube 6 zur Befestigung am schalenförmigen Aussenteil 2 führbar ist. Das Befestigungsteil 5a weist zudem eine Durchbrechung 5d auf, welche zur Aufnahme des Nocken 2c bestimmt ist. An der dem Dorn 5 gegenüberliegenden Stirnfläche 2a sind zwei vorstehende Nocken 2c angeordnet, wobei der Dorn 5 derart wählbar mit dem schalenförmigen Aussenteil 2 verbindbar ist, dass einer der Nocken 2c in der Durchbrechung 5d zu liegen kommt. Das Dornteil 5c ist bezüglich der beiden Durchbrechungen 5b zentriert angeordnet. Das Dornteil 5c könnte auch asymmetrisch, auf die eine Seite ausladend bezüglich dem Befestigungsteil 5a verlaufen. Dieser asymmetrische Dorn 5 kann derart ausgestaltet sein, dass er in zwei unterschiedlichen Lagen, nämlich um die Achse des Dornteils 5c um je 180 Grad gedreht, an der Stirnfläche 2a befestigbar ist.

Der Nocken kann auch am Befestigungsteil 3a, 4a, 5a vorstehend angeordnet sein, wobei in der Stirnfläche 2a eine entsprechende Aussparung zur Aufnahme des Nocken vorzusehen ist.

Das schalenförmige Aussenteil 2 sowie die Verankerungshilfsmittel 7 sind vorzugsweise aus Titan oder einer Titanlegierung gefertigt.

Der erfindungsgemässe Bausatz umfasst vorzugsweise eine Mehrzahl von unterschiedlich grossen schalenförmigen Aussenteilen 2 beziehungsweise auch unterschiedlich geformten Aussenteilen 2, beispielsweise mit oval oder rund verlaufender Stirnfläche 2a. Zudem umfasst der Bausatz eine Mehrzahl unterschiedlich geformter Verankerungshilfsmittel 7, welche alle ein Befestigungsteil 3a, 4a, 5a aufweisen, um das Verankerungshilfsmittel 7 an der Stirnfläche 2a fest mit dem schalenförmigen Aussenteil 2 zu verbinden. Die Verankerungshilfsmittel 7 können beispielsweise unterschiedlich lange Zungen aufweisen, oder unterschiedlich anatomisch vorgeformt sein. Zudem können die Verankerungshilfsmittel 7 auch für eine links oder rechts zu implantierende Aussenschale 1 ausgestaltet sein. Zur Verbindung sind vorzugsweise Schrauben 6, jedoch auch andere, eine Verbindung bewirkende Mittel geeignet. Die Stirnfläche 2a könnte auch eine andere Form aufweisen, und wie beispielsweise in Fig. 3 mit einem Ausschnitt eines Radialschnittes durch das Aussenteil 2 dargestellt, einen halbkreisförmigen Verlauf aufweisen. Die Befestigungsteile 3a, 4a, 5a müssten natürlich eine dem Verlauf der Stirnfläche 2a angepasst verlaufende Gegenfläche aufweisen.

Während dem Implantieren der Aussenschale 1 kann der Chirurg entsprechend dem Zustand des Beckenknochens die geeigneten Komponenten zusammenstellen. Zuerst wird ein geeignetes schalenförmiges Aussenteil 2 ausgewählt und, falls erforderlich, der Beckenknochen derart bearbeitet, dass das schalenförmige Aussenteil 2 in der anatomisch richtigen Lage im Beckenknochen angeordnet werden kann. Daraufhin werden aus der Vielzahl verfügbarer Verankerungshilfsmittel 7 diejenigen ausgewählt, welche ein sicheres Verankern des schalenförmigen Aussenteils 2 im Beckenknochen gewährleisten. Zumindest eines der in den Figuren 1 und 2 dargestellten Verankerungshilfsmittel 7 wird verwendet, vorzugsweise jedoch zwei. Die Lasche 3 weist einen anatomisch vorgeformten Verlauf auf. Der Chirurg legt beispielsweise bei im Beckenknochen eingesetztem Aussenteil 2 die Lasche 3 an die Stirnfläche 2a an und überprüft, ob der Verlauf der Laschenteile 3d, 3e mit dem anatomischen Verlauf des Beckenknochens ungefähr übereinstimmt. Bei zu grosser Abweichung wird die Lasche 3 nochmals entfernt und ausserhalb des Körpers mit Hilfe eines Werkzeuges nachgeformt. Die Lasche 3 wird wiederum an der Stirnfläche 2a angelegt und entweder mit Schrauben 6 fest mit dem Aussenteil 2 verbunden, oder nochmals entfernt und nachgeformt. Ein Vorteil der vorstehenden Nocken 2c ist darin zu sehen, dass während diesem Anpassen die Lasche 3 in die Nocken 2c einrastet und somit die gegenseitige Lage von Lasche 3 und Stirnfläche 2a genau definiert ist. Es kann sich als vorteilhaft erweisen zusätzliche Hilfsmittel zu verwenden. An Stelle der fest mit dem Aussenteil 2c verbundenen Nocken 2c könnte beispielsweise auch ein lösbarer Nocken oder eine kurze Stange in die Bohrung 2b der Stirnfläche 2a eingeschraubt werden, wobei die Bohrungen 3b des Befestigungsteils 3a in diesen Nocken beziehungsweise diese Stange eingeführt werden, so dass die Lasche 3 bezüglich der Stirnfläche 2a beziehungsweise dem Aussenteil 2 sicher geführt ist. Nachdem die Lasche 3 mit einer Schraube 6 befestigt ist kann der Nocken beziehungsweise die Stange entfernt werden, und an dieser Stelle ebenfalls eine Schraube 6 eingeführt werden.

Der Dorn 5 wird üblicherweise zur Verankerung in den Beckenknochen eingeschlagen. Dabei wird der Dorn 5 zuerst eingeschlagen, um danach fest am Aussenteil 2 befestigt zu werden. Es ist jedoch auch möglich den Dorn 5 vorerst fest mit dem Aussenteil 2 zu verbinden, um danach den Dorn 5 zusammen mit dem Aussenteil 2 einzuschlagen.

Das Verankerungshilfsmittel 7 kann auch als ein Hacken ausgestaltet sein, welcher ein Befestigungsteil 3a, 4a, 5a aufweist.

Die Durchbrechungen 3b, 4b, 5b des Befestigungsteil 3a, 4a, 5a könnten in Verlaufsrichtung der Stirnfläche 2a auch länglich verlaufend ausgestaltet sein, um bei in die Bohrung 2b eingesetzter Schraube 6 eine geringe Relativbewegung zwischen dem Aussenteil 2 und dem Verankerungsmittel 7 in Verlaufsrichtung der Stirnfläche 2a zu ermöglichen. Durch ein festes Anziehen der Schraube 6 wird diese Relativbewegung nachträglich unterbunden.

## Patentansprüche

1. Bausatz einer Aussenschale (1) für eine künstliche Hüftgelenkpfanne, umfassend ein schalenförmiges Aussenteil (2) sowie ein als Lasche (3, 4), Dorn (5) oder Haken (6) ausgestaltetes Verankerungshilfsmittel (7), wobei das schalenförmige Aussenteil (2) an dessen Äquator eine Stirnfläche (2a) mit Bohrungen (2b) aufweist, und wobei jedes Verankerungshilfsmittel (7) ein Befestigungsteil (3a, 4a, 5a) aufweist, welches derart bezüglich der Stirnfläche (2a) angepasst ausgestaltet ist, dass das Befestigungsteil (3a, 4a, 5a) mittels einer in die Bohrung (2b) eingreifenden Schraube (6) am schalenförmigen Aussenteil (2) befestigbar ist.

2. Bausatz nach Anspruch 1, dadurch gekennzeichnet, dass die Stirnfläche (2a) als ebene Fläche ausgestaltet ist.

3. Bausatz nach einem der vorhergehenden Ansprüche, dadurch gekennzeichnet, dass die Lasche (3; 4) aus einem Befestigungsteil (3a; 4a) und einem insbesondere zungenförmigen Laschenteil (3d, 3e; 4c) besteht.

4. Bausatz nach Anspruch 3, dadurch gekennzeichnet, dass das Laschenteil (3d, 3e; 4c) im wesentlichen entsprechend der Anatomie des Beckenknochens vorgeformt ist.

5. Bausatz nach einem der Ansprüche 3 oder 4, dadurch gekennzeichnet, dass die Lasche (3; 4) zumindest zwei, im wesentlichen insbesondere parallel zueinander verlaufende Laschenteile (3d,3e) aufweist.

6. Bausatz nach einem der Ansprüche 3 bis 5, dadurch gekennzeichnet, dass die Verankerungshilfsmittel (7), insbesondere die Laschen (3; 4) verformbar ausgestaltet sind.

7. Bausatz nach einem der vorhergehenden Ansprüche, dadurch gekennzeichnet, dass die Stirnfläche (2a) ein vorstehender Nocken (2c) und das Befestigungsteil (3a, 4a, 5a) eine Ausnehmung (3g, 5b) zur Aufnahme des Nockens (2c) aufweist, oder dass das Befestigungsteil (3a, 4a, 5a) ein vorstehender Nocken (2c) und die Stirnfläche (2a) eine Ausnehmung zur Aufnahme des Nockens (2c) aufweist.

8. Bausatz nach einem der vorhergehenden Ansprüche, dadurch gekennzeichnet, dass die Verankerungshilfsmittel (7) aus Titan oder einer Titanlegierung bestehen.

9. Bausatz nach einem der vorhergehenden Ansprüche umfassend eine Mehrzahl unterschiedlich ausgestalteter Aussenschalenteile (2) und/oder eine Mehrzahl unterschiedlich ausgestalteter Verankerungshilfsmittel (7).

10. Hüftgelenkpfanne umfassend eine Aussenschale (1) nach einem der vorhergehenden Ansprüche.
